# FASCICULE DE BREVET EUROPEEN

(11) **EP 0 700 677 B1**
(45) Date de publication et mention de la délivrance du brevet: **19.03.1997**
(21) Numéro de dépôt: 95401843.8
(22) Date de dépôt: 04.08.1995
(51) Int. Cl.: A61K 7/46, A61K 7/48

(54) **Utilisation d'un composé végétal pour la fixation d'un parfum dans une composition cosmétique et/ou dermatologique**
Verwendung eines pflanzlichen Materials zur Fixierung von Parfums in kosmetischen und/oder dermatologischen Zusammensetzung
Use of a plant compound for the fixing of perfume in a cosmetic and/or dermatological composition

(30) Priorité: 08.09.1994 FR 9410764
(43) Date de publication de la demande: 13.03.1996
(73) Titulaire: L'OREAL, 75008 Paris (FR)
(72) Inventeur: Lebreton, Françoise, I-91440 Bures Sur Yvette (FR); Gagnebien, Didier, F-92320 Chatillon (FR)
(74) Mandataire: Lhoste, Catherine

(56) Documents cités:
- EP-A- 0 279 328
- EP-A- 0 345 075
- WO-A-92/16195
- BE-A- 673 630
- DE-A- 4 301 266
- PATENT ABSTRACTS OF JAPAN vol. 4 no. 54 (C-008) & JP-A-55 025456 (RES INST FOR PROD DEV) 23 Avril 1980,
- PATENT ABSTRACTS OF JAPAN vol. 9 no. 17 (C-262) & JP-A-59 164711 (HIDEAKI TOUYAMA) 24 Janvier 1985,

## Description

La présente invention se rapporte à l'utilisation d'un composé végétal pour la fixation et/ou la libération prolongée d'un parfum dans une composition cosmétique et/ou dermatologique. Cette composition peut être utilisée pour le traitement thérapeutique et/ou pour le soin de la peau, aussi bien du visage que du corps ou des cheveux, le nettoyage de la peau et/ou des cheveux, le maquillage de la peau, tout en assurant leur parfumage.

On rappelle qu'un parfum est l'association de différentes substances odorantes. Chaque parfum présente une note de tête qui est l'odeur plus ou moins volatile diffusant en premier lors de l'application ou de l'ouverture du récipient le contenant, une note moyenne qui correspond au parfum complet (émission pendant quelques heures après la note de tête) et une note de base qui est la note finale la plus persistante (émission pendant plusieurs heures après la note moyenne).

L'être humain a de tout temps cherché à se parfumer, à parfumer les objets qui l'entourent ou les lieux dans lesquels il se trouve, et ceci, aussi bien pour masquer des odeurs peu agréables que pour donner une bonne odeur et ainsi avoir une satisfaction olfactive.

Il existe notamment un certain nombre de produits ou compositions desquels émanent des odeurs parfois fortes et/ou désagréables et qui nécessitent l'incorporation de parfum. Or, selon la nature de ces produits ou compositions, il n'est pas toujours aisé, d'y incorporer du parfum, et/ou d'en conserver les effets olfactifs agréables.

Ainsi, le document JP-A-03121169 enseigne la persistance d'un parfum dans une encre d'imprimerie grâce à l'utilisation d'une poudre à pouvoir adsorbant important, telle que du charbon actif imprégnée de parfum.

Malheureusement, pour obtenir de l'encre parfumée, il est nécessaire d'utiliser un matériau adsorbant, solide, qu'il faut enlever avant emploi de l'encre, ce qui entraîne une manipulation malaisée et un alourdissement du procédé d'utilisation.

Par ailleurs, il est également connu par le document JP-A-01101399 un détergent solide, contenant du parfum qui persiste au cours du temps et qui se libère progressivement au cours des différentes utilisations du détergent. Ce dernier est obtenu en incorporant d'abord le parfum dans une argile minérale composite organique puis en mélangeant l'argile parfumée à la composition détergente.

Malheureusement, l'utilisation d'une argile pour inclure un parfum dans cette composition détergente favorise la dégradation du parfum au contact de la chaleur ou d'agent alcalin présent dans la composition.

Il est en outre connu du document WO-A-8504803 l'emploi d'acide hyaluronique ou de l'un de ses sels dans une composition de parfum pour donner une meilleure rémanence à cette dernière.

Malheureusement, cet acide présente un pouvoir fixateur du parfum encore insuffisant. En outre, sa fonction acide peut provoquer des irritations lors de sa mise en contact avec la peau.

On connait également par le document WO-A-92/16195 que des cristaux liquides à base de polysaccharides peuvent encapsuler et délivrer des parfums. Toutefois, ces composés ne permettent pas la fixation suffisante du parfum.

Par ailleurs, il est aussi connu par la demande de brevet JP-A- 55 025456 d'utiliser la peau intérieure d'orange dans une base pour libérer lentement un parfum. Cependant, cet extrait végétal n'est pas suffisamment efficace pour fixer correctement le parfum.

En outre, le document DE-A-4301266 décrit une composition parfumée contenant un silicate pour l'adsorption réversible de l'huile parfumée, et le document EP-A-345075 décrit une émulsion eau/huile/eau gélifiée, le gélifiant pouvant contenir une gomme de xanthane.

Aussi, il subsiste le besoin d'une composition cosmétique et/ou dermatologique parfumée ne présentant pas les inconvénients ci-dessus et notamment, qui ne nécessite pas l'utilisation de composés solides pour incorporer le parfum dans la composition et/ou augmenter sa rémanence sans que ce dernier ne se dégrade à la longue, notamment au contact des autres constituants de la composition.

La demanderesse a découvert de façon surprenante que l'introduction d'un composé végétal dans une composition parfumée permet au parfum de ne pas se dégrader ainsi que de persister plusieurs heures sur le sujet ou objet à parfumer.

Cette composition permet notamment une libération prolongée dans le temps du parfum lors de l'utilisation de cette composition. En outre, malgré les ouvertures successives du flacon contenant la composition, cette dernière ne se dégrade pas et, en particulier, conserve une émission de la note de base du parfum jusqu'à épuisement du flacon.

A cet effet, l'invention porte sur l'utilisation dans une composition cosmétique et/ou dermatologique d'un composé végétal comme fixateur et/ou libérateur prolongé de parfum, le composé végétal comprenant au moins un extrait végétal non filmogène et/ou non épaississant et au moins une gomme végétale.

L'invention a également pour objet l'utilisation d'un composé végétal pour la fixation et/ou la libération prolongée d'un parfum dans une composition cosmétique et/ou dermatologique, ledit composé végétal comprenant au moins un extrait végétal choisi parmi les extraits de fucus, de lichen, de bourrache, d'amande, de guimauve, de lin et leurs mélanges.

L'extrait végétal non filmogène et/ou non épaississant peut être choisi parmi les extraits de fucus, de lichen, de bourrache, d'amande, de guimauve, de graine de lin, et leurs mélanges.

La gomme végétale peut être de préférence une gomme polysaccharidique. Celle-ci peut être choisie parmi la gomme de xanthane, de guar, d'alginate, d'algue, de cellulose, d'aguar-aguar, de caroube.

De façon préférentielle, le composé végétal est un mélange de gomme de xanthane et des extraits mentionnés précédemment, ci-après appelé mucilage.

Le fixateur de parfum peut être choisi en une quantité allant de 0,05 % et 10% en poids par rapport au poids total de la composition et est de préférence de 0,1 à 3% en poids par rapport au poids total de la composition.

La composition peut, en outre, comprendre un ou des solvants organiques comme les huiles (silicone), les alcools tels que l'éthanol mais aussi le propanol, l'isopropanol, des glycols et de façon général des alcools ayant de 2 à 8 atomes de carbone.

La présence de solvant dans une composition parfumée permet la libération massive de la note de tête par évaporation du solvant. Or, il est nécessaire que ce dernier soit présent en une faible quantité pour éviter que cette note de tête ne libère une trop importante quantité de parfum en une seule fois.

La présence d'un composé végétal comme fixateur de parfum dans la composition permet de prolonger la durée d'émission du parfum lors de l'utilisation de cette composition parfumée et par conséquent d'en prolonger la durée de la note de base.

Ainsi, pour qu'une composition parfumée présente une note de tête correcte et une note de base suffisante, il est souhaitable d'avoir un équilibre entre les quantités de solvant et de fixateur de parfum.

Ce rapport peut être compris entre 0,5 et 10 et peut être de préférence de 1 à 3 et par exemple de 2.

Il est également souhaitable d'avoir un rapport entre le parfum et le fixateur de parfum allant de 0,5 à 50 et de préférence de 1 à 30.

La composition selon l'invention comprend également au moins un composé support choisi parmi les supports classiquement utilisés dans le domaine cosmétique et/ou dermatologique. Elle peut en outre renfermer un ou plusieurs des actifs classiquement utilisés dans ces domaines, des gélifiants, des conservateurs, des charges, des émulsionnants

Ainsi la composition selon l'invention peut se présenter sous forme d'une émulsion huile-dans-eau ou eau-dans-huile (H/E ou E/H), d'un gel, d'une solution.

En particulier, la composition selon l'invention peut se présenter sous forme d'une crème de soin, d'une lotion nettoyante, d'un mascara, d'un fond de teint.

La présente invention se rapporte également à une utilisation d'un composé végétal pour la fixation et/ou la libération prolongée d'un parfum dans une composition cosmétique destinée au nettoyage de la peau, le composé végétal comprenant au moins un extrait végétal non filmogène et/ou non épaississant et au moins une gomme végétale.

L'invention a également pour objet l'utilisation d'un composé végétal pour la fixation et/ou la libération prolongée d'un parfum dans une composition cosmétique et/ou dermatologique, ledit composé végétal comprenant au moins un extrait végétal choisi parmi les extraits de fucus, de lichen, de bourrache, d'amande, de guimauve, de lin et leurs mélanges.

La description qui suit se réfère aux figures annexées dans lesquelles :

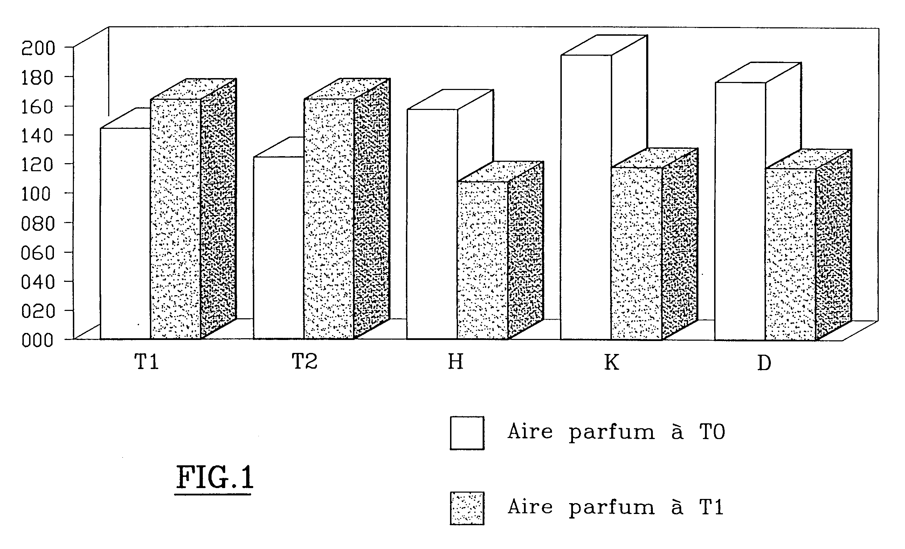

- la figure 1 donne sous forme d'histogrammes, la quantité de parfum évaporée pour différentes compositions, l'axe des abscisses désigne la nature des produits et l'axe des ordonnées désigne la surface intégrée par un calculateur, cette surface est exprimée en unité d'intégration (unité arbitraire).

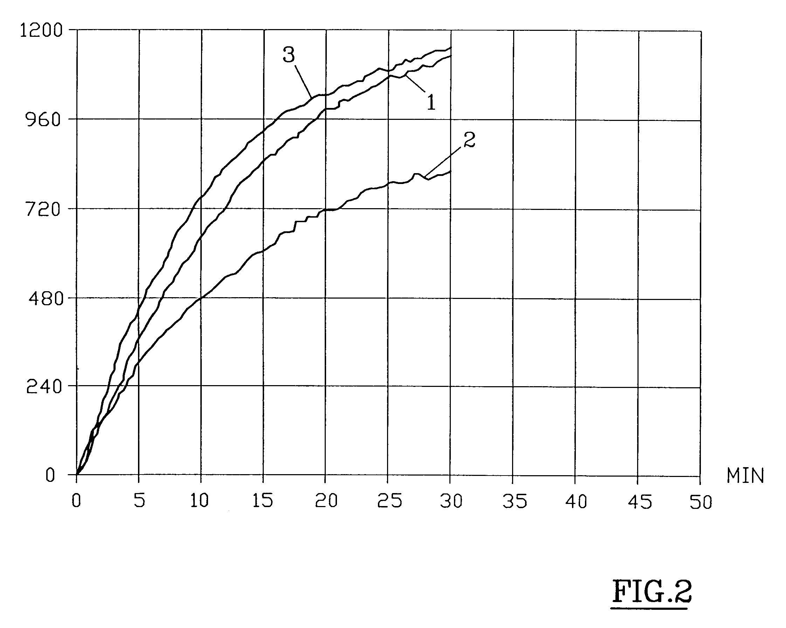

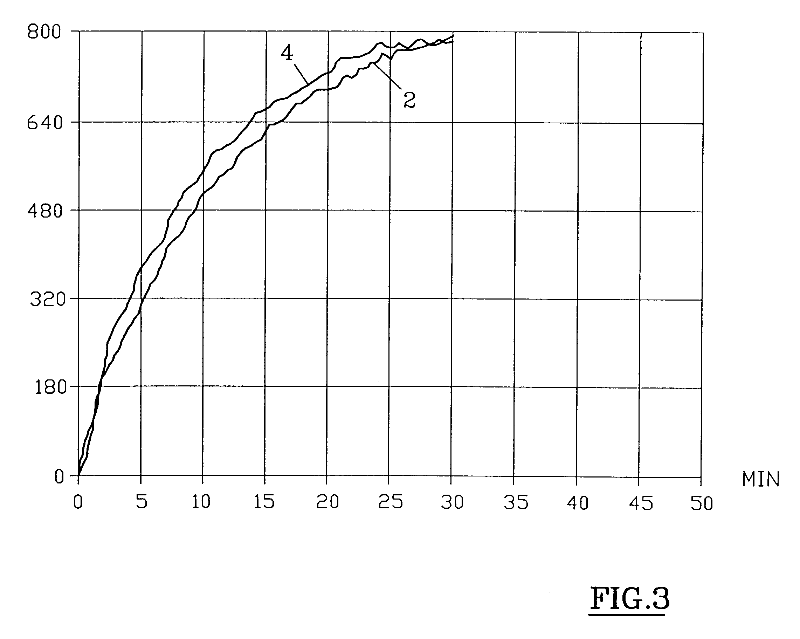

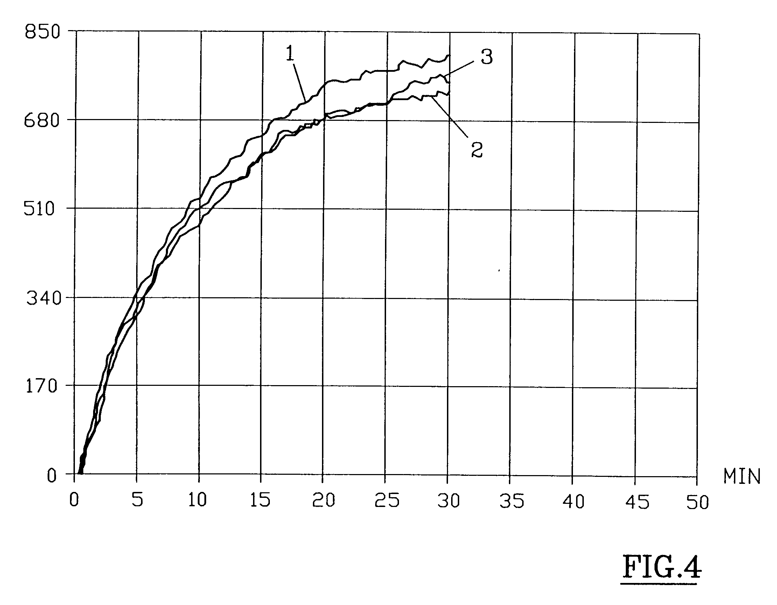

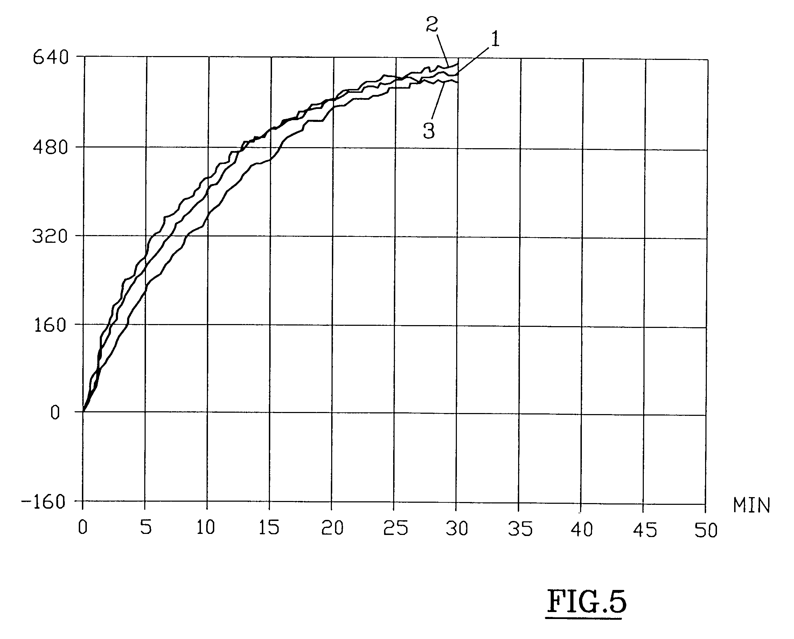

- les figures 2 à 5 donnent des courbes montrant l'évaporation naturelle du parfum pour différentes compositions ; l'axe des abscisses représente le temps en minutes et celui des ordonnées la concentration de composés volatils évaporés en unité arbitraire les courbes des figures 2 et 3 sont établies au temps T0 et celles des figures 4 et 5 sont établies respectivement après une et deux journées de conservation des compositions à l'air libre.

Les exemples de composition ci-après sont donnés à titre illustratif et dont les quantités sont données en pourcentage pondéral.

### Exemple

| *Phase grasse* | |
|---|---|
| - Huile de paraffine | 8 |
| - Huile de silicone (solvant) | 6 |

| *Emulsionnant* | |
|---|---|
| - Tensio-actif non ionique | 3 |

| *Phase aqueuse* | |
|---|---|
| - Mucilage | 3 |
| - Glycérine | 3 |
| - Conservateur | 0,6 |
| - Gélifiant | 0,35 |
| - Filtre U.V. | 0,05 |
| - Parfum | 4,5 |
| - Eau qsp | 100 |

Le lait obtenu est une émulsion huile-dans-eau. C'est un lait douche pour le nettoyage du corps. Ce lait libère du parfum pendant son utilisation et continue d'en libérer pendant plusieurs heures après application sur la peau.

### Première étude comparative

Cette première étude a pour but de montrer l'effet rémanent du parfum dans une composition selon l'invention. Elle est effectuée en comparant l'émission de parfum des compositions parfumées différentes suivantes :
- les compositions T1 et T2 sont des compositions selon l'invention contenant de l'éthanol (0,5 %), un parfum (4,5 %) et du mucilage (3 %),
- la composition H est une composition sans fixateur de parfum, contenant un parfum (4,5 %) et de l'éthanol (0,5 %);
- la composition K est une composition sans solvant, contenant un parfum (4,5 %) et de la gomme de xanthane (0,009 %);
- la composition D est une composition avec seulement du parfum (4,5 %).

Cette étude a été menée au moyen d'un chromatographe de type "Desorption - Concentration - Introduction" (DCI) telle que la platine DCI de la Société DELSY. C'est un système qui permet d'échantillonner des composés volatils dans un chromatographe. Cet appareil réalise des mesures dynamiques de différences de tension de vapeur. Ces mesures sont effectuées en quelques heures et sont fonction de la température ambiante et du taux d'humidité de l'atmosphère. Elles sont portées sur la figure 1. Cette figure montre les différentes valeurs de calcul de surface des différentes compositions à des temps T0 et T1.

Des échantillons de 300µl sont incorporés dans des nacelles en acier inoxydable. Les mesures prises au temps T0 (figure 1) correspondent à une évaporation pendant 5 minutes à température ambiante, et celles prises au temps T1 (figure 1), à une évaporation contrôlée pendant 1h30 à 40°C en étuve ventilée. Le temps de piégeage, ou de mesure, des échantillons à T0 est de 1 minute et à T1 de 5 minutes. Le temps de 5 min à T1 permet d'avoir une lecture fiable en concentration des composés volatils.

Pour chaque échantillon, on enregistre la quantité des principaux constituants de la phase vapeur, piégés au temps T0 et au temps T1.

De la figure 1, on en dégage les résultats suivants.

L'émission de parfum à T0 pour les compositions T1 et T2 est légèrement inférieure à celle des compositions H, K et D. Ceci est dû à une évaporation initiale du parfum (note de tête) plus élevée pour H, K et D que pour T. Ceci montre l'effet de rétention du composé végétal vis-à-vis du parfum.

Ensuite au temps T1, l'émission du parfum complet (note moyenne et note finale) dans les compositions T1 et T2 est supérieure à celle des formules H, K et D. Ceci montre l'effet rémanent du parfum dans la composition selon l'invention qui comprend un composé végétal comme fixateur de parfum.

En effet, les compositions qui ne comprennent pas de fixateur de parfum ( H, K et D) émettent à T1, quantitativement beaucoup moins de parfum que celle n'en contenant pas. La note de tête ayant été très importante au départ, à T0 pour H, K et D, a donc affaibli l'émission de la note moyenne et encore plus, celle de la note finale.

### Deuxième étude comparative

Cette seconde étude qui confirme les résultats de la première, est réalisée au moyen d'un olfactomètre du type "Alabaster". C'est un système muni d'un mono-capteur à semi conducteur (oxyde métallique SnO₂). Cette étude utilise les mêmes compositions T1, H, K et D que la première étude.

Cet appareil permet de mesurer quantitativement l'évaporation naturelle, à température ambiante, des composés volatils de chaque composition. Cette mesure est effectuée sur une période de 30 minutes en enceinte isolée.

Les résultats obtenus sont rassemblés sur les figures 2, 3, 4 et 5.

Les courbes de ces figures représentent pour chaque composition la quantité évaporée en unité arbitraire des composés volatils totaux, sur 30 minutes.

Les figures 2 et 3 rassemblent les courbes d'évaporation des composés volatils des compositions au temps T=0. Les figures 4 et 5 rassemblent les courbes d'évaporation des composés volatils des compositions, respectivement, après un et deux jours de conservation à l'air libre.

Pour chaque figure, la courbe 1 représente l'évaporation des composés volatils de la composition H, la courbe 2 celle des composés volatils de la composition K, la courbe 3 celle des composés volatils de la composition T et la courbe 4 celle des composés volatils de la composition D. Il faut noter que dans ce type de courbes, plus la pente à l'origine est grande, moins le parfum est rémanent.

La figure 2 montre bien qu'au temps T=0, la composition K (sans solvant mais avec de la gomme de xanthane) comporte nettement moins de composés volatils en comparaison avec les autres compositions T et H. Ceci est dû au fait que ces deux dernières compositions contiennent plus de solvant que la composition K.

La composition T émet d'abord plus de composés volatils que la composition H (pente plus forte). Au bout de 30 min, le phénomène diminue et l'extrapolation des courbes montre qu'il y a un effet de rétention du parfum dans la composition T puisque la courbe 1 passe au-dessus de la courbe 3.

Par ailleurs, la figure 3 montre à T0 que l'évaporation des composés volatils des compositions K et D sont comparables et très faibles. Ceci montre que la gomme présente dans la composition ne joue aucun rôle à T0, dans l'évaporation du parfum.

La figure 4 montre que la pente de la composition T est plus faible que celle de la composition H. Après 30 min, les courbes se rapprochent et l'extrapolation montre que la courbe 3 (composition T) va dépasser la courbe 1 (composition H) et, donc qu'après un certain temps, I'émission de parfum de la composition T sera supérieure à celle de la composition H.

Le fixateur de parfum joue d'abord le rôle d'un limitateur d'évaporation de parfum et, ensuite permet au parfum de se libérer au cours du temps. C'est ce que montre les figures 2 à 5.

En comparant les courbes de la figure 5, entre elles, on constate que la composition T émet quantitativement moins de parfum que les compositions H et K tout en restant à un niveau de détection pour le nez, suffisant, ce qui va dans le sens d'une rémanence du parfum plus élevée pour la composition T que pour les compositions K et H. L'émission de parfum après deux jours de conservation des compositions H et K, et par conséquent la quantité totale de parfum évaporé, est plus élevée que celle de T (même analyse que pour la figure 4 avec moins d'écart entre les courbes du fait que les composés volatils diminuent au cours du temps. Il devient difficile de les mesurer).

Aussi malgré une évaporation élevée de parfum à T0 par la composition T de l'invention, I'émission de parfum se prolonge dans le temps jusqu'à plus de deux jours et ce de façon détectable "olfactivement" parlant.

En comparant les courbes des figures 4 et 5 avec celles de la figure 2, on constate que l'émission de composés volatils après un et deux jours, est inférieure à celle des composés volatils à T0 (valeurs limites respectives, 840 et 630 au lieu de 1150).

L'émission de parfum des compositions H et T, après deux jours, rejoint celle de la composition K à T0, ce qui va dans le sens de la présence d'une grande quantité de parfum.

De plus, le rôle du solvant est devenu négligeable dans l'émission de parfum après un puis deux jours de conservation.

## Revendications

1. Utilisation d'un composé végétal pour la fixation et/ou la libération prolongée d'un parfum dans une composition cosmétique et/ou dermatologique, ledit composé végétal comprenant au moins un extrait végétal non épaississant et/ou non filmogène et au moins une gomme végétale.

2. Utilisation selon la revendication 1, caractérisée en ce que l'extrait végétal est choisi parmi les extraits de fucus, de lichen, de bourrache, d'amande, de guimauve, de lin et leur mélange.

3. Utilisation selon la revendication 1, caractérisée en ce que la gomme végétale est une gomme polysaccharidique.

4. Utilisation selon l'une quelconque des revendications précédentes, caractérisée en ce que la gomme végétale est la gomme de xanthane.

5. Utilisation d'un composé végétal pour la fixation et/ou la libération prolongée d'un parfum dans une composition cosmétique et/ou dermatologique, ledit composé végétal comprenant au moins un extrait végétal choisi parmi les extraits de fucus, de lichen, de bourrache, d'amande, de guimauve, de lin et leurs mélanges

6. Utilisation selon l'une des revendications précédentes, caractérisée en ce que le composé végétal est un mélange de gomme de xanthane et des extraits de fucus, de lichen, de bourrache, d'amande, de guimauve, de lin.

7. Utilisation selon l'une des revendications précédentes, caractérisée en ce que la composition contient, en outre, au moins un solvant volatil choisi parmi l'éthanol, l'isopropanol, les glycols.

8. Utilisation selon la revendication 7, caractérisée en ce que le rapport entre le solvant volatil et le composé végétal va de 0,5 à 10.

9. Utilisation selon la revendications 8, caractérisée en ce que le rapport entre le solvant volatil et le composé végétal est de 2.

10. Utilisation selon l'une des revendications 1 à 8, caractérisée en ce que le rapport entre le parfum et le composé végétal va de 0,5 à 50.

11. Utilisation selon l'une des revendications précédentes, caractérisée en ce que le composé végétal est présent dans une quantité allant de 0,05 à 10 % en poids par rapport au poids total de la composition.

12. Utilisation selon l'une des revendications précédentes, caractérisée en ce que le composé végétal est présent en une quantité allant de 0,1 à 3 % en poids par rapport au poids total de la composition.

13. Utilisation selon l'une des revendications précédentes, caractérisée en ce que la composition est une composition pour le nettoyage de la peau.

14. Utilisation d'un composé végétal pour la fixation et/ou la libération prolongée d'un parfum dans une composition cosmétique destinée au nettoyage de la peau, le composé végétal comprenant au moins un extrait végétal non filmogène et/ou non épaississant et au moins une gomme végétale.

## Claims

1. Use of a plant compound for the fixing and/or prolonged release of a perfume in a cosmetic and/or dermatological composition, the said plant compound comprising at least one non-thickening and/or non-film-forming plant extract and at least one plant gum.

2. Use according to Claim 1, characterized in that the plant extract is chosen from fucus, lichen, borage, almond, marshmallow and linseed extracts and mixtures thereof.

3. Use according to Claim 1, characterized in that the plant gum is a polysaccharide gum.

4. Use according to any one of the preceding claims, characterized in that the plant gum is xanthan gum.

5. Use of a plant compound for the fixing and/or prolonged release of a perfume in a cosmetic and/or dermatological composition, the said plant compound comprising at least one plant extract chosen from fucus, lichen, borage, almond, marshmallow and linseed extracts and mixtures thereof.

6. Use according to one of the preceding claims, characterized in that the plant compound is a mixture of xanthan gum and fucus, lichen, borage, almond, marshmallow and linseed extracts.

7. Use according to one of the preceding claims, characterized in that the composition contains, in addition, at least one volatile solvent chosen from ethanol, isopropanol, glycols.

8. Use according to Claim 7, characterized in that the ratio between the volatile solvent and the plant compound ranges from 0.5 to 10.

9. Use according to Claim 8, characterized in that the ratio between the volatile solvent and the plant compound is 2.

10. Use according to one of Claims 1 to 8, characterized in that the ratio between the perfume and the plant compound ranges from 0.5 to 50.

11. Use according to one of the preceding claims, characterized in that the plant compound is present in a quantity ranging from 0.05 to 10 % by weight relative to the total weight of the composition.

12. Use according to one of the preceding claims, characterized in that the plant compound is present in a quantity ranging from 0.1 to 3 % by weight relative to the total weight of the composition.

13. Use according to one of the preceding claims, characterized in that the composition is a composition for cleaning the skin.

14. Use of a plant compound for the fixing and/or prolonged release of a perfume in a cosmetic composition for cleaning the skin, the plant compound comprising at least one non-film-forming and/or non-thickening plant extract and at least one plant gum.

## Patentansprüche

1. Verwendung eines pflanzlichen Materials zur Fixierung und/oder protrahierten Freisetzung eines Parfums in einer kosmetischen und/oder dermatologischen Zusammensetzung, wobei das pflanzliche Material mindestens einen nicht verdickend wirkenden und/oder nicht filmbildenden Pflanzenextrakt und mindestens ein pflanzliches Gummi umfaßt.

2. Verwendung nach Anspruch 1, dadurch gekennzeichnet, daß der Pflanzenextrakt unter Fucusextrakt, Flechtenextrakt, Borretschextrakt, Mandelextrakt, Eibischextrakt und Leinextrakt sowie deren Gemischen ausgewählt ist.

3. Verwendung nach Anspruch 1, dadurch gekennzeichnet, daß das pflanzliche Gummi ein Polysaccharidgummi ist.

4. Verwendung nach einem der vorhergehenden Ansprüche, dadurch gekennzeichnet, daß das pflanzliche Gummi Xanthangummi ist.

5. Verwendung eines pflanzlichen Materials zur Fixierung und/oder protrahierten Freisetzung eines Parfums in einer kosmetischen und/oder dermatologischen Zusammensetzung, wobei das pflanzliche Material mindestens einen Pflanzenextrakt umfaßt, der unter Fucusextrakt, Flechtenextrakt, Borretschextrakt, Mandelextrakt, Eibischextrakt und Leinextrakt sowie deren Gemischen ausgewählt ist.

6. Verwendung nach einem der vorhergehenden Ansprüche, dadurch gekennzeichnet, daß das pflanzliche Material ein Gemisch aus Xanthangummi und Fucusextrakt, Flechtenextrakt, Borretschextrakt, Mandelextrakt, Eibischextrakt und/oder Leinextrakt ist.

7. Verwendung nach einem der vorhergehenden Ansprüche, dadurch gekennzeichnet, daß die Zusammensetzung ferner mindestens ein flüchtiges Lösungsmittel enthält, das unter Ethanol, Isopropanol und den Glykolen ausgewählt ist.

8. Verwendung nach Anspruch 7, dadurch gekennzeichnet, daß das Verhältnis von flüchtigem Lösungsmittel zu pflanzlichem Material 0,5 bis 10 beträgt.

9. Verwendung nach Anspruch 8, dadurch gekennzeichnet, daß das Verhältnis von flüchtigem Lösungsmittel zu pflanzlichem Material gleich 2 ist.

10. Verwendung nach einem der Ansprüche 1 bis 8, dadurch gekennzeichnet, daß das Verhältnis von Parfum zu pflanzlichem Material 0,5 bis 50 beträgt.

11. Verwendung nach einem der vorhergehenden Ansprüche, dadurch gekennzeichnet, daß das pflanzliche Material in einem Mengenanteil von 0,05 bis 10 Gew.-%, bezogen auf das Gesamtgewicht der Zusammensetzung, vorliegt.

12. Verwendung nach einem der vorhergehenden Ansprüche, dadurch gekennzeichnet, daß das pflanzliche Material in einem Mengenanteil von 0,1 bis 3 Gew.-%, bezogen auf das Gesamtgewicht der Zusammensetzung, vorliegt.

13. Verwendung nach einem der vorhergehenden Ansprüche, dadurch gekennzeichnet, daß die Zusammensetzung eine Zusammensetzung zur Reinigung der Haut ist.

14. Verwendung eines pflanzlichen Materials zur Fixierung und/oder protrahierten Freisetzung eines Parfums in einer kosmetischen Zusammensetzung, die zur Reinigung der Haut bestimmt ist, wobei das pflanzliche Material mindestens einen nicht filmbildenden und/oder nicht verdickend wirkenden Pflanzenextrakt und mindestens eine pflanzliches Gummi umfaßt.
